# EUROPÄISCHE PATENTSCHRIFT

(11) **EP 0 236 698 B1**
(45) Veröffentlichungstag und Bekanntmachung des Hinweises auf die Patenterteilung: **16.06.1993**
(21) Anmeldenummer: 87100757.1
(22) Anmeldetag: 21.01.1987
(51) Int. Cl.: A61B 17/56

(54) **Schraubendreher für die Chirurgie**
Screwdriver for surgery
Tournevis à usage chirurgical

(30) Priorität: 22.01.1986 DE 3601715
(43) Veröffentlichungstag der Anmeldung: 16.09.1987
(73) Patentinhaber: Heinl, Thomas, Dr., D-32427 Minden (DE)
(72) Erfinder: Heinl, Thomas, Dr., D-32427 Minden (DE)
(74) Vertreter: Pöhner, Wilfried Anton, Dr.

(56) Entgegenhaltungen:
- EP-A- 0 048 038
- WO-A-82/01645
- DE-U- 8 528 003
- DE-U- 8 528 004
- FR-A- 924 131
- GB-A- 1 277 105
- US-A- 2 518 155
- US-A- 3 488 779
- US-A- 3 498 351
- US-A- 3 554 251
- US-A- 3 695 321
- US-A- 3 842 825
- US-A- 4 190 091
- THE KLOMAN INSTRUMENT CO., INC., 1954 Edition Katalog, Seiten F-112,F-114; Washington, US "Equipment and supplies for hospitals and physicians."

## Beschreibung

Die Erfindung betrifft einen Schraubendreher für die Chirurgie insbesondere zum Verbinden von Knochenfragmenten (Osteosynthese) gemäß dem Oberbegriff des Anspruchs 1.

In der Chirurgie ist allgemein geläufig, die durch Unfälle oder operative Eingriffe erzeugten Knochenfragmente unter anderem dadurch zu fixieren, daß sie mit Verbindungsplatten verschraubt werden. Gegenüber anderen, grundsätzlich denkbaren Vorgehensweisen empfiehlt sich das Verschrauben vor allen Dingen dann, wenn die einzelnen Knochenfragmente sehr stabil und präzise zueinander ausgerichtet positioniert werden müssen, wie dies beispielsweise bei Schädel-, Gesichts-, Wirbel- oder Handfrakturen in besonderem Maße zutrifft. Nach erfolgter Verheilung der einzelnen Knochenfragmente miteinander werden bei nicht resorbierbaren oder weniger verträglichen Materialien durch einen erneuten chirurgischen Eingriff die Platten entfernt. Zur Implantation werden im Stande der Technik ebene Platten mit äquidistanten Bohrungen angeboten, die bei entsprechendem Bedarf während der Operation durch ein entsprechendes Instrumentarium zumindest teilweise gebogen und den individuellen Verhältnissen angepaßt werden müssen. Die Befestigung erfolgt über Schlitzschrauben üblichen Aufbaus, die ihrerseits über aus dem Maschinenbau allgemein bekannten Schraubendreher eingebracht werden. Wie keiner näheren Erläuterung bedarf, ergibt sich eine wesentliche Verlängerung der Operationszeit daraus, daß während der Operation die einzelnen Platten angepaßt und z. B. gebogen werden müssen und daß beim Hantieren mit Schrauben und Schraubendrehern die große Gefahr besteht, so daß ständig darauf geachtet werden muß, daß die unmittelbar vor dem Eindrehen in den Knochen auf dem Schraubendreher befindliche Schraube herunterfällt und in der Operationswunde verschwindet, was zu erheblichen zusätzlichen Komplikationen führt. Es versteht sich von selbst, daß hier wie bei allen anderen Operationen die Operationszeit selbst zur Reduzierung der Belastung des Patienten möglichst gering gehalten werden muß.

Aus dem Stande der Technik, beispielsweise der US-PS 3 498 351 sind Schraubendreher bekannt, bei denen auf der Klinge koaxial und gegen Feder- oder Magnetkraft verschieblich eine Spannzange mit am unteren Ende radial und federnd bewegbaren Klemmbacken angebracht und auf der Spannzange eine axial verschiebbare Überwurfhülle federnd zu befestigt ist, die in ihrer unteren Position die Klemmbacken nach innen preßt. Vereinfacht ausgedrückt soll auf der Klinge eine Schraubenhaltevorrichtung angebracht werden, die ihrerseits aus einer Spannzange und einer Überwurfhülle gebildet wird Mit Hilfe der an der Spannzange befindlichen Klemmbacke kann der Kopf einer Schraube erfaßt werden und, dadurch daß die Überwurfhülle in Richtung auf die Schraube zu axial verschoben wird, kann der Kopf der Schraube festgelegt und in das entsprechende Knochenfragment eingeschraubt werden. Wird die Überwurfhülle nach hinten, also von der Schraube weg bewegt, werden die Klemmbacken freigegeben, so daß sie sich federnd nach außen bewegen und hierdurch den Schraubenkopf frei geben.
Unabhängig von der Position der Überwurfhülle und damit zusammenhängend der Klemmbacken steht der Schraubendreher über die Klinge mit der Schraube in Verbindung, so daß ein weiteres Eindrehen der Schrauben möglich ist und eine maximale Eindrehtiefe deshalb erreicht werden kann, da auch der normalerweise von den Klemmbacken umfaßte Schraubenkopf bis in die Platte oder das Knochenfragment hinein bewegt werden kann. Die vorgeschlagene Konstruktion ermöglicht damit eine größere Eindrehtiefe als sie bei Instrumenten möglich wären, die während des gesamten Eindrehvorganges den Kopf umgeben und festhalten würden. Zur besseren Führung und Vermeiden des Verlustes der Schraube wird mit Hilfe der Klemmbacken an der Spannzange der Schraubenkopf zunächst gleichmäßig und zirkulär erfaßt und ihr hierdurch ein fester und sicherer Halt verliehen. Mit Erreichen einer gewissen Eindrehtiefe wird der Schraubenkopf freigegeben und die Schraube selbst nurmehr noch mit der Klinge des Schraubendrehers weiterbewegt, um auf diese Weise eine maximale Eindrehtiefe zu erlangen. Durch die federnde Ausbildung der Klemmbacken und insbesondere der federnden Anordnung der Überwurfhülle auf der Spannzange wird zusätzlich sichergestellt, daß die Überwurfhülle nicht ohne weiteres z. B. unter Einwirkung der Schwerkraft nach vorne fallen kann, da zur Überwindung der Federkräfte ein gewisser Kraftaufwand erforderlich ist, der, sofern er über dem Eigengewicht der Überwurfhülle liegt, ein vertikales Arbeiten auch bei zurückgefahrener Überwurfhülle möglich macht. Hinzu muß allerdings kommen, daß die Spannzange gegen Federkraft auf der Klinge verschieblich ist.

Besonders einfach und rasch arbeiten läßt sich, wenn die Spannzange nur so weit über die Klinge maximal verschiebbar ist, daß die Klemmbacken den Schraubenkopf erfassen und die Überwurfhülle nicht über die Spannzange hinaus verschiebbar ist. Die Spannzange kann dann nach vorne bis zum Anschlag bewegt werden und sie befindet sich dann in der exakten Position zur Erfassung der Schrauben. Nachdem die Klemmbacken über den Kopf geschoben sind, werden sie durch Bewegung der Überwurfhülle bis zum Anschlag federnd nach innen auf die Schraube zu bis zur Anlage bewegt. Würden Spannzange oder Überwurfhülle noch weiter nach vorne bewegbar sein, müßte beim Erfassen des Schraubenkopfes die Spannzange präzise eingestellt werden und die Überwurfhülle würde beim Arbeiten die Einsicht auf das Gewinde der Schraube verdecken.
Dennoch ist als nachteilig anzusehen, daß während der Drehbewegung ein Umgreifen der Hand erfolgen muß, was in aller Regel eine Beeinträchtigung der Ausrichtung der Schraube zur Folge hat.

Hiervon ausgehend hat sich die Erfindung die Weiterentwicklung derartiger Schraubendreher dahingehend zur Aufgabe gemacht, daß der Eindrehvorgang bei exakter Ausrichtung der Schraube und gleichzeitiger Möglichkeit hoher Kraftübertragung möglich ist.

Gelöst wird diese Aufgabe erfindungsgemäß dadurch, daß der Handgriff des Schraubendrehers geteilt und beide Teile rotationsmäßig und axial gegebeinander bewegbar sind so daß der eine Teil des Handgriffes mit einigen der Finger einer Hand festgehalten werden kann, während die Rotationsbewegung des anderen Teils zum Einbringen und Herausdrehen der Schrauben einzig durch Bewegung der anderen Fingern derselben Hand vorgenommen werden kann, darüberhinaus kann die axiale Bewegbarkeit ein Festlegen oder Lösen der beiden Teile mittels Druck oder Zugausübung ermöglichen. Es ist dann einerseits möglich, den Griff und damit den gesamten Schraubendreher mit dem einen Teil festzuhalten und durch Rotation des anderen schnell und bequem eine Schraube ein- oder herauszudrehen. Es gelingt ein exaktes Ausrichten beim Ein- und Ausdrehen der Schraube. Da die den einen Teil des Handgriffes umgreifende Hand raumfest und die Rotationsbewegungen des anderen einzig durch Bewegung der Finger vorgenommen wird, ist weiterhin ausgeschlossen, daß sich der Operationshandschuh um den Handgriff wickelt. Die axiale Verschiebbarkeit ermöglicht, daß bei axialer Druck- oder Zugausübung die beiden Teile sich relativ zueinander bewegen, insbesondere ineinandergreifen können und sich miteinander verbinden. Insbesondere beim Lösen der Schrauben ist es wichtig einen hohen Anpressdruck der Klinge zu erreichen und gleichzeitig ein hohes Drehmoment ausüben zu können. Dies wird dadurch möglich, daß bei einem großen Anpreßdruck die beiden Teile ineinandergreifen und demzufolge gemeinsam von der ganzen Hand ergriffen und ein wesentlich größeres Drehmoment aufgebracht werden kann.

Zur Verbesserung und Erleichterung der Reinigung, Desinfektion und Sterilisation des erfindungsgemäßen Schraubendrehers sind vorteilhaft Klinge, Spannzange, Überwurfhülle voneinander trenn- und zerlegbar und die Klinge ist in einen Schnellverschluß einsetzbar. Der Schnellverschluß kann in einer der an sich bekannten Weisen ausgebildet sein und eine Sechskantaufnahme für die Klinge besitzen. Ein zusätzlicher Vorteil ist in der Austauschbarkeit der einzelnen Elemente bei Defekten oder Einsatz unterschiedlicher Arbeitslängen und Klingendurchmesser zu sehen. Schließlich ist noch die Aufnahme der Klinge, also z.B. der Schnellverschluß drehbar im Handgriff gelagert. Dies erlaubt das Ausführen von Drehbewegungen der Klinge bei gleichzeitig raumfestem Griff. In der Praxis wird man mit den hinteren Fingern der Hand den Griff festhalten und die zum Einbringen der Schrauben erforderliche Drehbewegung mit den vorderen Fingern durch die Rotation der Klingenaufnahme, die deshalb von außen zugänglich sein muß, vornehmen. Der Vorteil besteht darin, daß unter Beibehaltung der axialen Position der Schraube und mit einer Hand das Eindrehen erfolgen kann. Ein rasches und präzises Positionieren der Schraube im jeweiligen Knochenfragment ist die Folge. Empfehlenswert ist, die Aufnahme der Klinge durch Zug oder Druck festzulegen und zu blockieren, damit das volle von der gesamten Hand ausübbare Drehmoment übertragen werden kann.

Eine weitere Möglichkeit besteht darin, an der Klinge des Schraubendrehers mittig einen in axialer Richtung weisenden Stift anzuformen und Schlitzschrauben mit axialer Bohrung zu verwenden und einzusetzen. Der an der Klinge des Schraubendrehers befindliche Stift ist dann so bemessen, daß er in die axiale Bohrung eingreift, so daß sich gegenüber der Handhabung einer Schlitzschraube zusätzliche Verbesserungen der Führungseigenschaften vor allem in axialer Richtung und eine hohe transversale Stabilität erreichen läßt. Selbstverständlich ist eine Abstimmung oder Dimensionierung des Stiftes in Bezug auf die in der Schraube befindliche axiale Bohrung erforderlich.

Zur Verwendung im Zusammenhang mit dem soeben erläuterten Schraubendreher werden Schlitzschrauben mit axialer Bohrung versehen, deren Tiefe größer/gleich der des Schlitzes ist und/oder deren Durchmesser größer als die Breite des Schlitzes ist. Der an der Klinge des Schraubendrehers befindliche Stift greift in die axiale Bohrung und erzeugt eine hohe transversale Stabilität, eine optimale Kraftübertragung und Friktion sowie eine bessere Führung in axialer Richtung, so daß das Arbeiten wesentlich erleichtert und darüber hinaus auch das Herunterfallen der Schraube von der Klinge verhindert wird. Das gleiche Ziel erreicht man, wenn der Durchmesser der Bohrung größer als die Breite des Schlitzes ist.

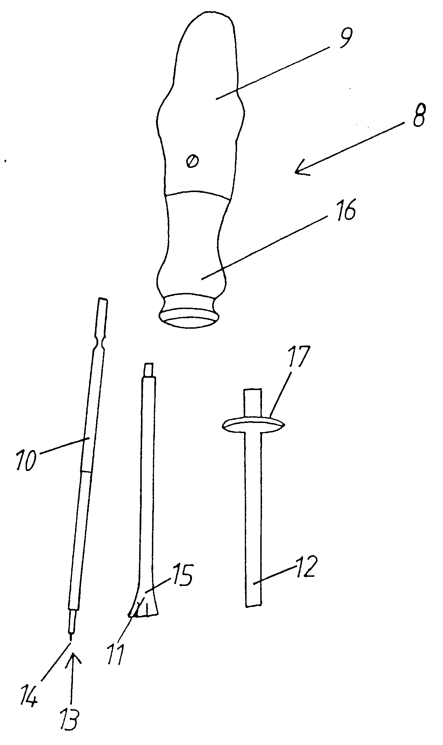

Weitere Einzelheiten, Merkmale und Vorteile der Erfindung lassen sich dem nachfolgenden Beschreibungsteil entnehmen, in dem anhand der Zeichnung ein Ausführungsbeispiel des erfindungsgemäßen Schraubendrehers in zerlegtem Zustand näher erläutert wird.

In der Figur ist ein erfindungsgemäßer Schrauhenzieher 8 zur besseren Verdeutlichung in zerlegtem Zustand widergegeben. Dieser besteht in seinem grundsätzlichen Aufbau aus einem Handgriff 9, einer Klinge 10, einer Spannzange 11 und einer Überwurfhülle 12. Dabei ist die Klinge 10 von üblichem Aufbau, besteht also aus einem vollzylindrischen Stab und einer Spitze 13. In Abweichung hiervon ist zusätzlich und etwa in der Mitte ein in axialer Eichtung weisender Stift 14 angeformt, der in eine entsprechende, axial verlaufende Bohrung der hierdurch zu befestigenden und nicht abgebildeten Schraube eingreift.
Auf diese Klinge 10 wird koaxial eine im wesentlichen hohlzylindrisch geformte Spannzange 11 aufgeschoben, die an ihrem unteren Ende mit radial bewegbaren und federnd angebrachten Klemmbacken 15 ausgerüstet ist. In dem Bereich der Klemmbacken 15 weist die Spannzange 11 einen etwas größeren Durchmesser auf. Wird nun die Überwurfhülle 12, die ein Hohlzylinder ist, aufgebracht, muß sie vom oberen Ende her aufgeschoben werden und kann auf Grund des inneren Durchmessers der Überwurfhülle 12 nicht über den Klemmbacken 15 hinausgeschoben werden.
Der Handgriff 9 ist von üblichem Aufbau und an seinem vorderen Ende mit einer drehbaren Aufnahme 16 für die Klinge 10 ausgerüstet. Der Zusammenbau geschieht in der Weise, daß zunächst die Spannzange 11 auf die Klinge 10 und darüber die Überwurfhülle 12 geschoben wird und die hierdurch gebildete Gesamtheit durch Einschieben der Klinge 10 in die Aufnahme 16 mit dem Griff 8 verbunden wird. Die Drehbarkeit der Aufnahme 16 gewährleistet, daß bei festgehaltenem Handgriff 9 eine Drehbewegung der Klinge 10 vorgenommen werden kann.

Die Benutzung geschieht in der Weise, daß bei zurückgefahrener Spannzange 11 und Überwurfhülle 12 die Klinge 10 mit ihrer Spitze 13 und dem Stift 14 auf den Kopf einer entsprechenden Schraube aufgesetzt und bei zurückgezogener Überwurfhülle 12 die Spannzange 11 damit bei geöffneten Klemmbacken 15 soweit nach vorne geschoben wird, bis der hier nicht dargestellte Schraubenkopf umfaßt wird. Dann wird beispielsweise mit Hilfe des Tellers 17 die Überwurfhülle 12 nach vorne geschoben und hier durch die Klemmbacken 15 nach innen bewegt und der Schraubenkopf festgelegt. Damit entstehen kraftschlüssige Verbindungen zwischen dem Schraubenkopf und der Innenfläche der Klemmbacken 15 sowie deren Außenfläche und der Überwurfhülle 12. Des Schraubgewinde ist nach außen hin überstehend und bleibt gut einsehbar. Nun kann das untere Ende der Schraube in einer entsprechenden Bohrung 2 einer Platte eingeführt und unter axialem Druck gegen den Handgriff 9 durch Drehbewegungen der Aufnahme 16 eingeschraubt werden und zwar soweit, bis die Klemmbacken nahezu anzuliegen kommen.
Durch Zurückziehen der Überwurfhülle 12 bewegen sich die Klemmbacken 15 wieder nach außen, geben den Schraubenkopf frei, so daß nurmehr noch mit Hilfe der Klinge 10 und vermittels des Stiftes 14 das weitere Eindrehen der Schraube bis zur endgültigen Tiefe erfolgt. Dabei sind sowohl Überwurfhülle 12 als auch Spannzange 11 so federnd gelagert, daß beide im zurückgefahrenen Zustand, also bei geöffneten Klemmbacken 15, nicht von sich aus bewegbar sind, so daß auch bei vertikaler Stellung der Klinge 10 ein Arbeiten möglich und ein Herunterfellen von Spannzange 11 und Überwurfhülle 12 unterbunden wird.

Im Ergebnis erhält man durch die Erfindung einen chirurgischen Schraubendrehen, mit dessen Hilfe ein wesentlich rascheres und auch präziseres Arheiten bei der Verschraubung von Knochenfregmenten (Osteosynthese) möglich wird.

## Patentansprüche

1. Schraubendreher (8) für die Chirurgie zum Verbinden von Knochenfragmenten (Osteosynthese) mit einer auf der Klinge (10) koaxial und gegen Feder- oder Magnetkraft verschiebliche Spannzange (11) mit am unteren Ende radial und federnd bewegbaren Klemmbacken (15), wobei auf der Spannzange (11) eine axial verschiebbare Überwurfhülle (12) federnd angeordnet ist, die in ihrer unteren Position die Klemmbacken (15) nach innen preßt und die Spannzange (11) maximal soweit über die Klinge (10) verschiebbar ist, daß die Klemmbacken (15) einen Schraubenkopf erfassen können und die Überwurfhülle (12) nicht über die Spannzange (11) hinaus verschiebbar ist, **dadurch gekennzeichnet**, daß der Handgriff (9) des Schraubendrehers (8) geteilt ist und beide Teile rotationsmäßig und axial gegeneinander bewegbar sind, so daß der eine Teil des Handgriffes mit einigen der Finger einer Hand festgehalten werden kann, während die Rotationsbewegung des anderen Teils zum Einbringen und Herausdrehen der Schrauben einzig durch Bewegung der anderen Fingern derselben Hand vorgenommen werden kann, darüberhinans kann die axiale Bewegbarkeit ein Festlegen oder Lösen der beiden Teile mittels Druck oder Zugausübung ermöglichen.

2. Schraubendreher nach Anspruch 1 oder 2, **dadurch gekennzeichnet**, daß im Handgriff (9) ein Rechts/Links-Ratschenmechanismus und/oder ein einstellbarer Drehmomentenbegrenzer untergebracht ist.

3. Schraubendreher nach einem der Ansprüche 1 bis 3, **dadurch gekennzeichnet**, daß die Klinge (10) in einen Schnellverschluß einsetzbar ist und/oder Klinge (10), Spannzange (11) und Überwurfhülle (12) zerlegbar sind.

4. Schraubendreher nach einem der Ansprüche 1 bis 4, **dadurch gekennzeichnet**, daß die Aufnahme (16) der Klinge, wie zum Beispiel der Schnellverschluß, drehbar und von außen zugänglich im Handgriff (9) gelagert und/oder festlegbar ist.

5. Schraubendreher nach einem der Ansprüche 1 bis 5, **dadurch gekennzeichnet**, daß an die Klinge (10) des Schraubendrehers (8) mittig ein in axialer Richtung weisender Stift (14) angeformt ist.

## Claims

1. Screw driver (8) for surgery to join bone fragments (osteosynthesis) having on blade (10) a collet chuck (11) which is movable coaxially against spring or magnetic force with clamping jaws (15) which are movable radially and in a resilient manner on the lower end, whereby on collet chuck (11) a coupling jacket (12) that is movable axially is disposed in a resilient manner which in its lower position presses clamping jaws (15) inwards and collet chuck (11) is at most movable over blade (10) so that clamping jaws (15) can grip a screw head and coupling jacket (12) is not movable beyond collet chuck (11), **wherein** handle (9) of said screw driven (8) is divided and both parts are movable axially and rotatively in relation to each other, so that one part of the handle can be held on to with a few fingers of one hand, while the rotational movement of the other part to insert and unscrew the screws is done solely by moving the other fingers on the same hand, over and above this the axial movement can enable both parts to be fixed or released by pressing or pulling.

2. Screw driver according to claim 1 or 2, **wherein** a right-hand/left-hand ratchet mechanism and/or an adjustable torque limiter is disposed in said handle (9).

3. Screw driver according to one of claims 1 to 3, **wherein** said blade (10) can be used in a quick action closure and/or blade (10), collet chuck (11) and coupling jacket (12) are dismountable.

4. Screw driver according to one of claims 1 to 4, **wherein** the reception (16) of said blade, for example said quick action closure, is rotatable and is mounted or can be fixed in said handle (9) so as to be accessible from outside.

5. Screw driver according to one of claims 1 to 5, **wherein** a pin (14) pointing in an axial direction is moulded centrically on to said blade (10) of said screw driver (8).

## Revendications

1. Tournevis chirurgical (8) pour réunir des fragments osseux (ostéosynthèse), avec une pince expansible (11) montée coaxiale sur la lame (10) et coulissant contre l'action d'un ressort ou d'un aimant, et munie à son extrémité inférieure de mors (15) mobiles radialement et de façon élastique, un manchon enveloppe (12) coulissant axialement étant monté de façon élastique sur la pince expansible (11), le manchon enveloppe comprimant dans sa position inférieure les mors (15) vers l'intérieur, la pince expansible (11) pouvant coulisser sur la lame (10), au plus jusgu'à ce que les mors (15) puisse enserrer une tête de vis, le manchon enveloppe (12) ne pouvant coulisser au-delà de la pince expansible (11),
**caractérisé en ce que**,
le manche (9) du tournevis (8) est divisé, les deux parties étant mobiles en rotation et en translation axiale l'une par rapport à l'autre, de sorte que l'une des parties du manche peut être maintenue de quelques doigts d'une main, le mouvement de rotation de l'autre des parties, destiné à mettre en place la vis et à la retirer, pouvant être obtenu par le seul jeu des autres doigts de la même main, la mobilité axiale pouvant de plus permettre, par l'exercice d'une pression ou d'un écartement, de fixer ou de désolidariser les deux parties.

2. Tournevis selon la revendication 1, **caractérisé en ce que**, dans le manche (9) est disposé un mécanisme à rochet droite/gauche et/ou un limiteur de couple ajustable.

3. Tournevis selon l'une des revendications 1 à 2, **caractérisé en ce que**, la lame (10) peut être mise en place dans une douille à fermeture rapide et/ou que la lame (10), la pince expansible (11) et le manchon enveloppe (12) sont démontables.

4. Tournevis selon l'une des revendications 1 à 3, **caractérisé en ce que**, la partie (16) qui reçoit la lame, telle que la douille à fermeture rapide, peut tourner et, avec accessibilité par l'extérieur, est supportée dans le manche et/ou y est immobilisable.

5. Tournevis selon l'une des revendications 1 à 4, **caractérisé en ce que**, la lame (10) du tournevis (8) est munie d'une pointe (14) disposée en son centre et orientée en direction axiale.
